# EUROPEAN PATENT APPLICATION

(11) **EP 2 308 340 A1**
(43) Date of publication of application: **13.04.2011**
(21) Application number: 09425393.7
(22) Date of filing: 07.10.2009
(51) Int. Cl.: A45D 34/02, A61L 9/12, B60H 3/00

(54) **Device for the treatment of environments**

(71) Applicant: Errebi S.r.l., 20124 Milano (MI) (IT); GOLD CONSULTING ITALIA DI GIORGI F.S.A.S., 20010 Vittuone (IT)
(72) Inventor: Giorgi, Fabrizio, 20010 Vittuone (MI) (IT); Restelli, Claudio, 20124 Milano (IT)
(74) Representative: Carangelo, Pierluigi

(57) **Abstract**

A device (1) for the treatment of environments comprises a support and holding structure (2), a cartridge seat (3) formed by the support and holding structure (2) and suitable to receive a cartridge (4) with treating substance, a securing portion (5) for the securing of the support structure (2) to a unit, wherein the securing portion (5) comprises magnetic means (6).

## Description

The object of the present invention is a device for the treatment of environments, for example, through the emission of a volatile perfuming substance or a disinfectant, absorbing substance.

Devices for the treatment of environments through the emission of a volatile substance are known, comprising a support and holding structure with a seat to receive a treating substance, for example, a tablet or a vial containing a perfume for environments or a substance suitable to absorb odours or polluting substances contained in the environmental air, means for the gradual release of the treating substance into the environment, and an coupling portion, particularly a mechanical hook, for the securing of the support and holding structure to a unit, for example, to a piece of furniture, a casing, an appliance, a sanitary fixture, a shower box, a wall, the interior of a closet, a curtain, etc.

The devices for the treatment of environments of the prior art, while being satisfactory from the point of view of the environmental air treatment, have the drawback that the coupling portion is not sufficiently versatile to fit well the various shape and material characteristics of the uses to which it is desired to secure the device. In fact, due to the securing difficulties or to improvised securing modes, particularly on units with irregular shapes or in materials that are soft, slippery, or frequently wet, the devices for the treatment of environments frequently come off and are often projecting and bulky, and very rarely result to be well inserted in the aesthetical context of the unit itself.

In order to obviate the securing difficulties and the blemishes deriving from improvised securing modes, the user often chooses to place the environments treatment devices in hidden positions, and therefore at least partially covered, thus compromising a regular emission of the treating substances or a free access of the environmental air to a purifying substance that is supported or dispensed by the treatment device.

Therefore, the object of the present invention is to provide a device for the treatment of environments of the above-specified type, having such characteristics as to be easily securable to units with a different shape and material, without however compromising the contact between the treating substance and the environmental air and negatively affecting the aesthetics of the unit or environment of application.

These and other objects are achieved by a device for the treatment of environments comprising:
- a support and holding structure;
- a cartridge seat formed by the support and holding structure and suitable to receive a cartridge with treating substance;
- a securing portion for the securing of the support and holding structure to a unit;
in which such securing portion comprises magnetic means.

Thanks to the magnetic means, it is possible to secure the treatment device to various units, independently from the shape of the device itself, as well as the shape and material of the unit, thus obviating the overall dimensions and blemishes of mechanical hooks or the destructive action of adhesives on delicate surfaces.

In accordance with an aspect of the present invention, the magnetic means comprise two magnets that are mutually attracted and configured so as to exert a clamping force.

This advantageously allows clamping the treating device to a unit and clamping at the same time the unit to another object or hold the unit tight, as in the case of a curtain stop device for the treatment of the environmental air according to the present invention.

In accordance with a further aspect of the present invention, the magnetic means are configured to exert a permanent pressure on the cartridge with treating substance, so as to trigger and/or promote the emission thereof.

In accordance with a further aspect of the present invention, the magnetic means are positioned relative to the cartridge seat, so as to expose the cartridge to a magnetic field, and the cartridge is magnetically susceptible so as to trigger and/or promote the emission of the treating substance or the treatment (for example, purification) of the environmental air.

Thanks to the above-mentioned characteristics, the cartridges with treating substance can be in a substantially inactive state when they are outside the cartridge seat and not subjected to the pressure and/or the magnetic field generated by the magnetic means and activated through the insertion in the cartridge seat and the magnetic securing of the treatment device to the unit.

In accordance with a further aspect of the present invention, the magnetic means comprise at least one permanent magnet received by a magnet seat of the support and holding structure, in which such magnet seat completely wraps the magnet, thus insulating it from the external environment and/or from the cartridge with treating substance. In order to better understand the present invention and appreciate the advantages thereof, some exemplary, non-limiting embodiments thereof will be described herein below, with reference to the Figures, in which:

Fig. 1 is a schematized front view of a device for the treatment of environments according to an embodiment of the invention;

Fig. 2 is a sectional view of the device in Fig. 1;

Fig. 3 is an enlarged view of the detail III in Fig. 2;

Fig. 4 is a schematized sectional view of a treatment device according to a further embodiment;

Fig. 5 is a front view of a treatment device according to a further embodiment;

Fig. 6 is a sectional view according to the line VI-VI in Fig. 5.

Fig. 7 is an exploded view of a treatment device according to an embodiment;

Fig. 8 is a perspective view of the treatment device of Fig. 7 in an assembled configuration.

With reference to the Figures, a device for the treatment of environments (hereinafter termed "treatment device") is generally indicated with the numeral reference 1. The treatment device 1 comprises a support and holding structure 2, a cartridge seat 3 formed by the support and holding structure 2 and suitable to receive a cartridge 4 with treating substance, as well as a securing portion 5 for the securing of the support structure 2 to a unit.

In accordance with an aspect of the invention, the securing portion 5 comprises magnetic means 6, in particular, at least one permanent magnet.

Thanks to the magnetic means 6, it is possible to secure the treatment device 1 to various units, independently from the shape of the treatment device 1 itself, as well as the shape and material of the unit, thus obviating the overall dimensions and the blemishes of mechanical hooks or the destructive action of adhesives on delicate surfaces.

Within the scope of the present disclosure, by the term "cartridge 4" is meant a support bearing and containing the treating substance, for example, a deodorant or a purifying substance suitable to cover, neutralize or absorb odours and polluting agents in the environmental air.

The cartridge 4 can comprise a single solid body, for example, a tab or a disc or a porous and absorbing granulate, a liquid, or a gel, one or more absorbing wood or adsorbing pumice stone bodies, porous and absorbing synthetic material.

In accordance with an embodiment, the cartridge 4 is configured as a disposable cartridge that can be removably received in the cartridge seat 3, allowing the replacement thereof after the exhaustion of the treating substance contained therein.

Alternatively, the whole treating device 1 is configured as a disposable device, in which the cartridge 4 with treating substance is irreversibly integrated to the support structure 2.

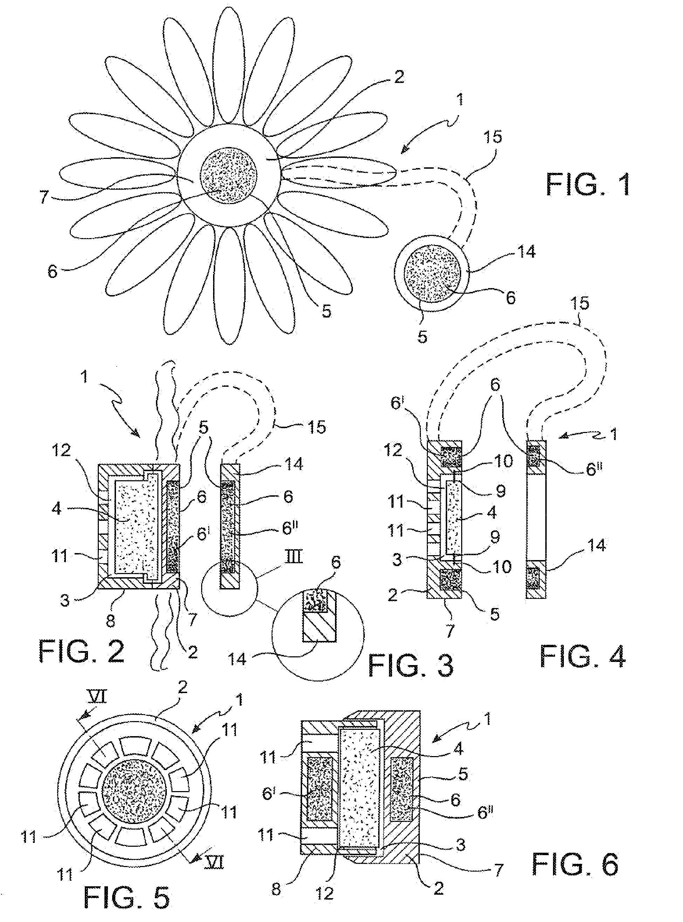

In accordance with an embodiment (Fig. 2), the support structure 1 has a base body 7 and a lid 8 that is removably connectable to the base body 7, internally defining together the above-mentioned cartridge seat 3.

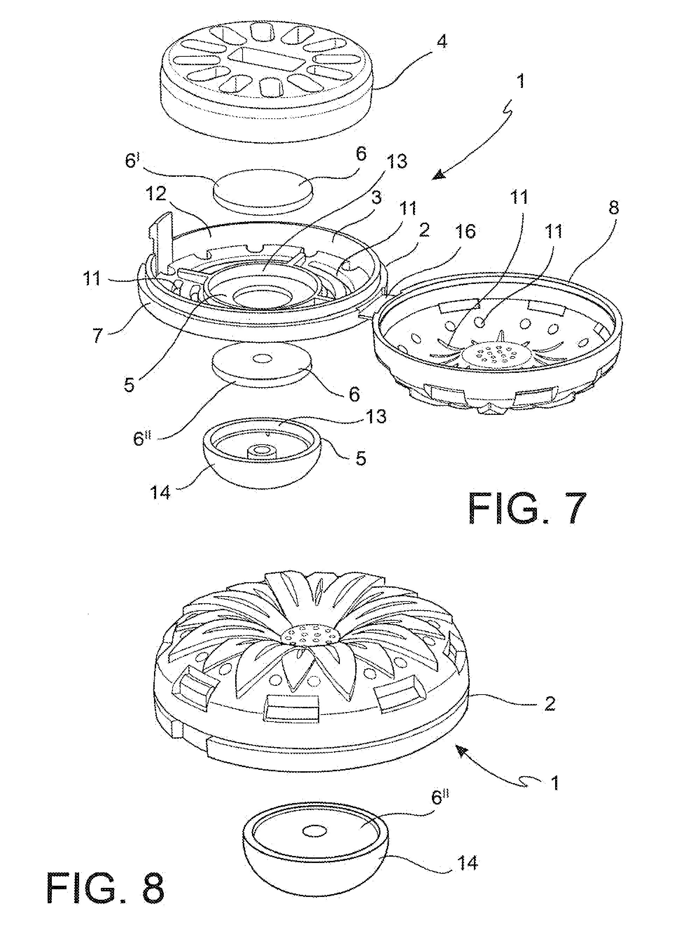

The connection between the base body 7 and the lid 8 can be a snap connection (through an elastic snap tooth), an interference connection, or a screw connection, and the lid 8 can be hinged to the base body 7, preferably through a flexure hinge 16 integrally connecting the base body 7 and the lid 8 (see, for example, Fig. 7).

In accordance with an embodiment (Fig. 4), the cartridge seat 3 comprises coupling means 9 suitable to engage corresponding counter-coupling means 10 formed in the cartridge 4 with treating substance, so as to receive and hold such cartridge 4 without having to close the cartridge seat 3 with a lid. Such coupling means 9 and counter-coupling means 10 can comprise snap portions, for example, elastic flat bands or teeth, threads, or bayonet portions.

Advantageously, the coupling means 9 comprise a magnet, and the counter-coupling means 10 of the cartridge 4 comprise a portion in ferromagnetic material or graphite that is attracted by such magnet. Preferably, but not necessarily, the coupling means 9 are formed by the magnetic means 6 of the treatment device 1 securing portion 5.

In accordance with a further aspect of the invention, the support and holding structure 2 comprises one or more windows or passages 11 that put an inner space 12 of the cartridge seat 3 into communication with the external environment, so as to allow the exchange between the treating substance and the surrounding air, while preventing an undesired direct contact with the cartridge 4, for example, in the presence of children or pets.

In accordance with a further aspect of the present invention, the magnetic means 6 comprise at least one permanent magnet received in a magnet seat 13 of the securing portion 5, in which such magnet seat 13 completely wraps the magnet, thus insulating it from the external environment and/or the cartridge seat 3 (see Figs. 2 and 3). This allows protecting the magnet in the case of a repeated repositioning of the treatment device 1 and allows protecting units having delicate surfaces against scratches due to a coarse magnetic material.

Alternatively, the magnet can be housed without any outer cover.

By way of example, the magnetic means themselves can comprise one or more disk-shaped (Figs. 1, 2, 5, 6) or annular (Fig. 4) permanent magnets. It shall be apparent that also other geometric shapes of the magnets are contemplated.

In accordance with a further aspect of the present invention, the securing portion 5 magnetic means 6 comprise two magnets 6', 6" or a magnet 6' and a ferromagnetic body 6" that are mutually attracted and configured so as to exert a clamping force.

This allow clamping the treating device 1 to a unit (for example, a curtain) and clamping at the same time the unit (the curtain) to another object or holding the unit tight, thus combining the functions of object-holder (ad es. curtain stop) and environmental air treatment in a single device.

The two magnetically attracted magnets or bodies 6', 6" are advantageously received in corresponding magnet seats 13 that are formed, the one integrally with the cartridge seat 3, and the other one in a distinct mobile portion 14 of the securing portion 5 (which in this case is composed of two pieces).

The magnetic securing mobile portion 14 can be completely separated or movably connected to the support structure 2, for example, through a belt 15, cable, or chain.

In accordance with a further aspect of the present invention (see, for example, Fig. 6), the magnetic means 6, the cartridge seat 3, and the cartridge with treating substance 4 are configured so that, in an actuation configuration, the magnetic means 6 exert a permanent pressure on the cartridge 4, so as to trigger and/or promote the emission of the treating substance.

To this aim, the lid 8 can be movable (writhing certain limits) relative to the base body 7 and, urged by the magnetic force of the magnetic means 6, press against the cartridge 4 received in the cartridge seat 3.

In accordance with a still further aspect of the present invention, the magnetic means 6 are positioned relative to the cartridge seat 3 so that, in an actuation configuration, the magnetic means 6 expose the cartridge 4 to a magnetic field, and the cartridge 4 is magnetically susceptible so as to trigger and/or promote the emission of the treating substance or the treatment (for example, purification) of the environmental air.

In this manner, the cartridges 4 with treating substance can be in a substantially inactive state when they are outside the cartridge seat and not subjected to the pressure and/or the magnetic field generated by the magnetic means, and activated through the insertion in the cartridge seat and the magnetic securing of the treatment device to the unit.

Alternatively, the emission of the treating substance will be able to take place also by simple dispersion in contact with the surrounding air.

Ornamental and camouflage portions are indicated with the numeral reference 16, for example, fabric flower petals, connected to the support structure 2.

The support and holding structure 2 itself will be able to be made of wood, metal materials, resins, plastic materials, board, rubber, fabric, polystyrol, glass.

The treatment device 1 can be advantageously used for the environmental air treatment, particularly in domestic environments, motor vehicles, boats, schools, public buildings, and offices. The environmental air treatment relates particularly, without limitation, to a treatment by a deodorizing perfume or odor absorber or insecticide.

It shall be apparent that to the device for the treatment of environments according to the present invention, those of ordinary skill in the art, to the aim of meeting specific, contingent needs, will be able to make further modifications and variations, all anyhow falling within the protection scope of the invention, as defined by the following claims.

## Claims

1. A device (1) for the treatment of environments, comprising:
- a support and holding structure (2);
- a cartridge seat (3) formed by the support and holding structure (2) and suitable to receive a cartridge (4) with treating substance;
- a securing portion (5) for the securing of the support structure (2) to a unit,
**characterized in that** the securing portion (5) comprises magnetic means (6).

2. The device (1) according to claim 1, comprising said cartridge (4), which has a support bearing said treating substance, selected from the group consisting of:
- a deodorant;
- a purifying substance suitable to cover, neutralize, or absorb odours and polluting agents.

3. The device (1) according to claim 1 or 2, wherein said cartridge (4) is removably and replaceably received in the cartridge seat (3).

4. The device (1) according to one of the claims 1 to 3, wherein the support structure 1 comprises a base body (7) and a lid (8) connectable to the base body (7) so as to internally define together said cartridge seat (3).

5. The device (1) according to claim 4, wherein the connection between the base body (7) and the lid (8) is a snap or interference connection.

6. The device (1) according to one of the claims 1 to 3, wherein the cartridge seat (3) comprises coupling means (9) suitable to engage corresponding counter-coupling means (10) formed in the cartridge (4) with treating substance, so as to hold said cartridge (4) in the cartridge seat (3)

7. The device (1) according to claim 6, wherein said coupling means (9) and said counter-coupling means (10) comprise snap portions.

8. The device (1) according to claim 6, wherein said coupling means (9) comprise a magnet, and said counter-coupling means (10) of the cartridge (4) comprise a portion in ferromagnetic material that is attracted by such magnet.

9. The device (1) according to claim 8, wherein said coupling means (9) are formed by the magnetic means (6) of said securing portion (5).

10. The device (1) according to any one of the preceding claims, wherein said support and holding structure (2) defines one or more passages (11) connecting an inner space (12) of the cartridge seat (3) to the external environment, so as to allow the exchange between the cartridge (4) treating substance and the surrounding air and to protect from direct contacts with the cartridge (4).

11. The device (1) according to any one of the preceding claims, wherein said magnetic means (6) comprise at least one permanent magnet received in a magnet seat (13) of the securing portion (5), wherein said magnet seat (13) completely wraps said permanent magnet, thus insulating it from the external environment.

12. The device (1) according to any one of the preceding claims, wherein said magnetic means (6) comprise two magnets (6', 6") or a magnet (6') and a ferromagnetic body (6") that are mutually attracted and configured so as to exert a clamping force.

13. The device (1) according to claim 12, wherein said two magnetically attracted magnets or bodies (6', 6") are received in corresponding magnet seats (13) formed the one integrally with the cartridge seat (3) and the other one in a distinct mobile portion (14) of the securing portion (5).

14. The device (1) according to any one of the preceding claims, wherein the magnetic means (6), the cartridge seat (3), and the cartridge (4) with treating substance are configured so that, in an actuation configuration, the magnetic means (6) exert a permanent pressure on the cartridge (4) to promote the emission of the treating substance.

15. The device (1) according to any one of the preceding claims, wherein the cartridge (4) is magnetically susceptible and said magnetic means (6) are positioned relative to the cartridge seat (3) so that, in an actuation configuration, the cartridge (4) is exposed to a magnetic field to promote the environmental air treatment.
